(19) Europäisches Patentamt
European Patent Office
Office européen des brevets

(11) Veröffentlichungsnummer : **0 530 613 B1**

(12) # EUROPÄISCHE PATENTSCHRIFT

(45) Veröffentlichungstag der Patentschrift : **25.01.95 Patentblatt 95/04**

(51) Int. Cl.$^6$ : **G03F 7/027,** C08F 20/36, C07D 229/00, C07C 275/62

(21) Anmeldenummer : **92114284.0**

(22) Anmeldetag : **21.08.92**

(54) **Bilderzeugungselement mit einem fotopolymerisierbaren Monomer.**

(30) Priorität : **03.09.91 DE 4129284**

(43) Veröffentlichungstag der Anmeldung : **10.03.93 Patentblatt 93/10**

(45) Bekanntmachung des Hinweises auf die Patenterteilung : **25.01.95 Patentblatt 95/04**

(84) Benannte Vertragsstaaten : **BE DE FR GB**

(56) Entgegenhaltungen :
**EP-A- 125 862**
**EP-A- 0 017 336**
**EP-A- 0 249 468**
**US-A- 3 622 320**
**US-A- 4 019 972**
**US-A- 4 145 544**

(73) Patentinhaber : **Agfa-Gevaert AG**
**Kaiser-Wilhelm-Allee**
**D-51373 Leverkusen (DE)**

(72) Erfinder : **Podszun, Wolfgang, Dr.**
**Roggendorfstrasse 55**
**W-5000 Köln 80 (DE)**
Erfinder : **Müller, Michael, Dr.**
**Richard-Zanders-Strasse 34**
**W-5060 Bergisch Gladbach 2 (DE)**
Erfinder : **Uytterhoeven, Herman, Dr.**
**Boslaan 6**
**B-2820 Bonheiden (BE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 530 613 B1

## Beschreibung

Die vorliegende Erfindung betrifft ein Bilderzeugungselement mit einem fotopolymerisierbaren Monomer der Formel I.

Die Verwendung der Fotopolymerisation zur Herstellung von Abbildungen durch bildmäßige Belichtung mit aktinischer Strahlung ist bekannt. Die Abbildungsmethoden beruhen auf dem Prinzip, eine Differenzierung der Eigenschaften der belichteten und nichtbelichteten Teile einer fotopolymerisierbaren Schicht zu bewirken, beispielsweise eine Differenzierung hinsichtlich Löslichkeit, Adhäsion, Leitfähigkeit, Brechungsindex, Klebrigkeit, Permeabilität oder Diffusionsfähigkeit von eindringenden Substanzen, wie z.B. Farbstoffen.

Abbildungssysteme, die auf einer Differenzierung der Löslichkeit beruhen, sind beispielsweise beschrieben in US-A-4 019 972 und EP-A-0 125 862.

Abbildungssysteme, die auf einer Differenzierung der Klebrigkeit beruhen, werden in den US-Patentschriften 3 060 024, 3 085 488 und 3 649 268 beschrieben. Entsprechend der in den US-Patentschriften offenbarten Methode verliert die fotopolymerisierbare Schicht im Bereich der bildmäßig belichteten Stellen ihre Klebrigkeit, während die unbelichteten Stellen klebrig bleiben. Die nicht belichteten Stellen können daher mit trockenen Pigmenten angefärbt werden, um das Bild sichtbar zu machen.

Weitere Abbildungsmethoden, die auf der Fotopolymerisation mit anschließender Trockenentwicklung beruhen, werden in den Patentschriften US-A-3 245 796, EP-A-0 362 827, US-A-4 587 198 und US-A-3 060 023 beschrieben.

Die Fotopolymerisation wird in vielfältiger Weise zur Bildwiedergabe genutzt. Dabei werden Methoden sowohl mit nasser als auch mit trockener Bildentwicklung angewendet. Letztere sind besonders anwendungsfreundlich und weisen entscheidende ökologische Vorteile auf.

Allerdings ist die mit trocken entwickelbaren fotopolymerisierbaren Zusammensetzungen erzielbare Auflösung relativ gering, und dies stellt ein besonderes Problem dar bei der Wiedergabe von fein gerasterten Bildern.

Der vorliegenden Erfindung liegt die Aufgabe zugrunde, ein Bilderzeugungselement bereitzustellen, das eine fotopolymerisierbare Zusammensetzung enthält, durch die Anwendung von Wärme entwickelbar ist und mit dem eine hohe Auflösung erzielt werden kann.

Gegenstand der Erfindung ist ein Bilderzeugungselement mit einer Schicht einer fotopolymerisierbaren Zusammensetzung, gekennzeichnet durch einen fotopolymerisierbaren Gehalt an mindestens einem Monomer der folgenden Formel I

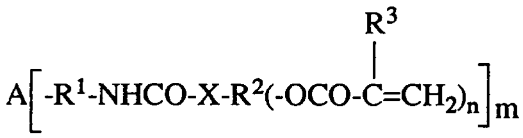 (I),

worin bedeuten

A einen zwei- oder dreibindigen kondensierten Harnstoffrest einer der Formeln

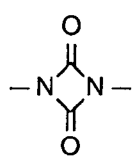 und 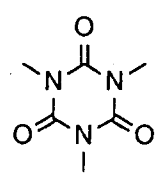 ;

X -O- oder -NR- mit R = $C_1$-$C_{12}$-Alkyl;

$R^1$ einen zweibindigen Kohlenwasserstoffrest mit 2 bis 25 C-Atomen;

$R^2$ einen (n + 1)-bindigen, linearen oder verzweigten, gegebenenfalls durch bis zu 3 O-Atome unterbrochenen Kohlenwasserstoffrest mit 2 bis 18 C-Atomen;

$R^3$ H oder Methyl;

n eine ganze Zahl von 2 bis 5; und

m 2 oder 3.

Der durch X dargestellte zweibindige Rest ist vorzugsweise Sauerstoff (-O-). Falls X für -NR- steht, ist R ein linearer oder verzweigter Alkylrest; Beispiele hierfür sind Methyl, Ethyl, Propyl und t-Butyl.

Der durch R[1] dargestellte Kohlenwasserstoffrest mit 2 bis 25 C-Atomen kann gegebenenfalls durch Sauerstoff unterbrochen sein. Es handelt sich hierbei um aliphatische, aromatische oder gemischt aliphatisch-aromatische Kohlenwasserstoffreste. Beispielsweise kann R[1] einen zweibindigen gradkettigen oder verzweigten aliphatischen Rest, bevorzugt mit 2 bis 12 C-Atomen, bedeuten. Beispiele hierfür sind die folgenden:

Ethylen, Propylen, 1,4-Tetramethylen, 1,6-Hexamethylen und 2,2,4-Trimethyl-1,6-hexamethylen sowie gegebenenfalls deren Isomere.

R[1] kann auch ein mono- oder polycyclischer, gesättigter oder aromatischer Kohlenwasserstoffrest mit 6 bis 24 C-Atomen, vorzugsweise mit 6 bis 14 C-Atomen, sein. Beispielsweise seien genannt:

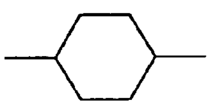 , 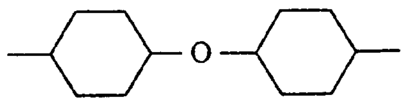 , 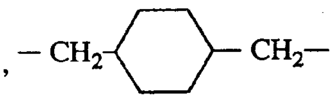 ,

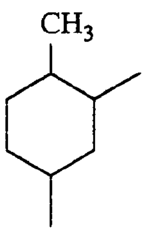 , 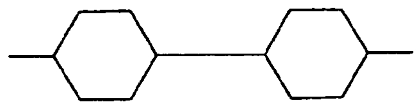 , 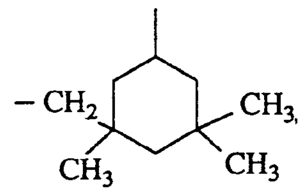

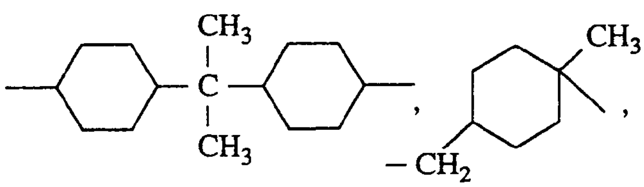 , 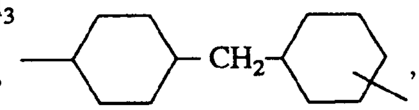 ,

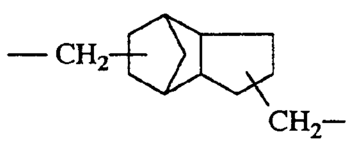

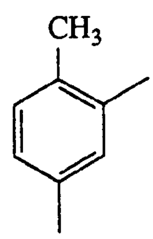 , 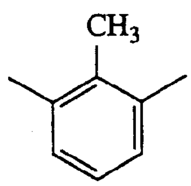 , 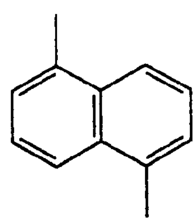 , 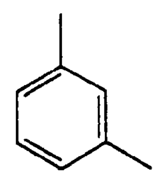 ,

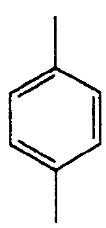 , 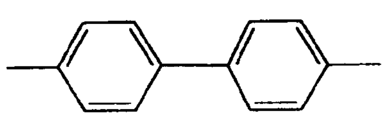 , 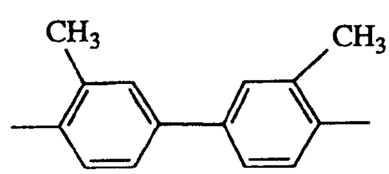 ,

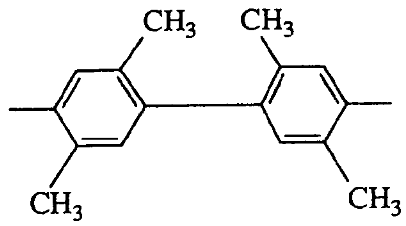 , 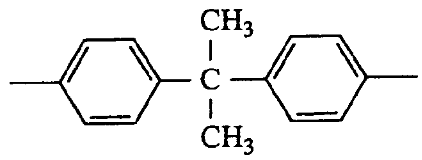 ,

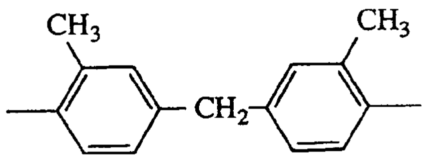 , 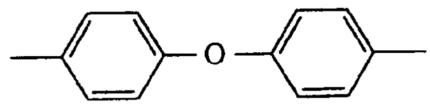 ,

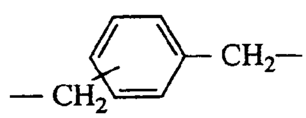 , 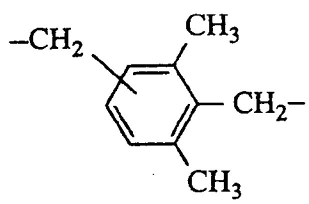 ,

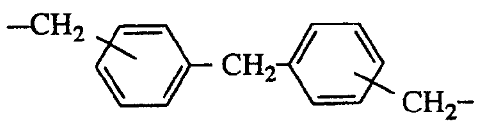 , 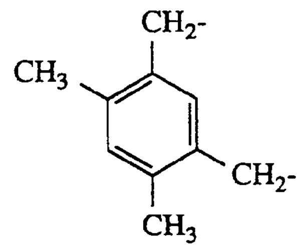 ,

Der durch $R^2$ dargestellte, gegebenenfalls durch O-Atome unterbrochene Kohlenwasserstoffrest ist vorzugsweise ein zwei- bis vierbindiger Rest mit 2 bis 12 C-Atomen. Beispiele hierfür sind:

Ethylen, Propylen und 1,2-Butylen; des weiteren kommen auch beispielsweise die folgenden mehrbindigen Reste infrage:

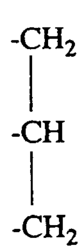 , 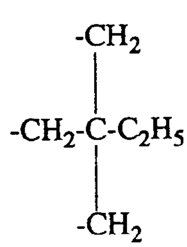 , 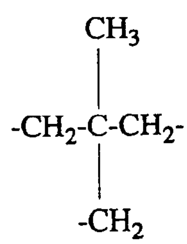 , 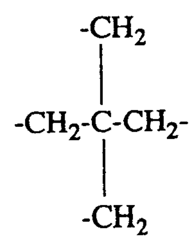 ,

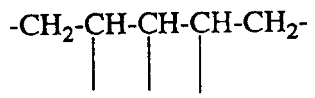
,
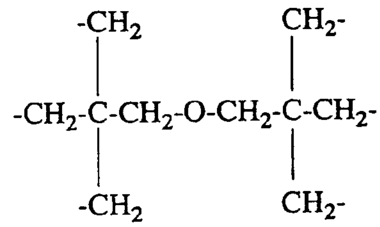
.

n ist vorzugsweise 2 oder 3.

Besonders bevorzugt ist ein Bilderzeugungselement mit einem Monomer der Formel I, worin bedeuten

X -O-;

$R^1$ einen aliphatischen oder cycloaliphatischen Rest mit 5 bis 8 C-Atomen;

$R^2$ einen drei- oder vierbindigen Kohlenwasserstoffrest (n = 2 oder 3) mit 3 bis 6 C-Atomen;

$R^3$ H oder Methyl.

Einige Verbindungen sind neu; Beispiele hierfür sind die nachfolgend angegebenen Monomere M-3, M-4, M-5 und.

Die in der nachfolgenden Tabelle 1 aufgeführten Verbindungen sind Beispiele von polyfunktionellen Monomeren, die für die vorliegende Erfindung besonders gut geeignet sind.

<u>Tabelle 1</u>

M-1

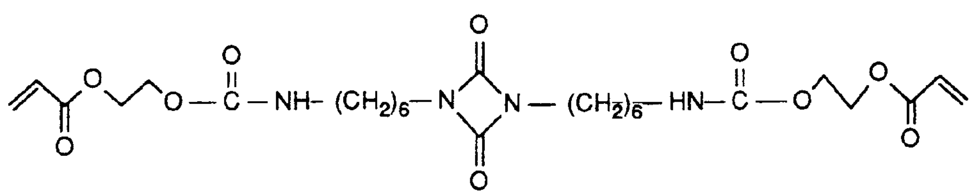

M-2

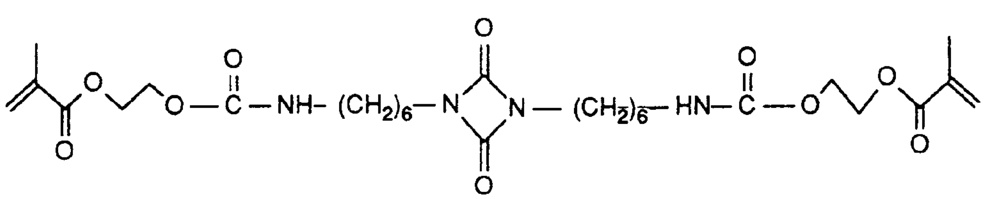

M-3

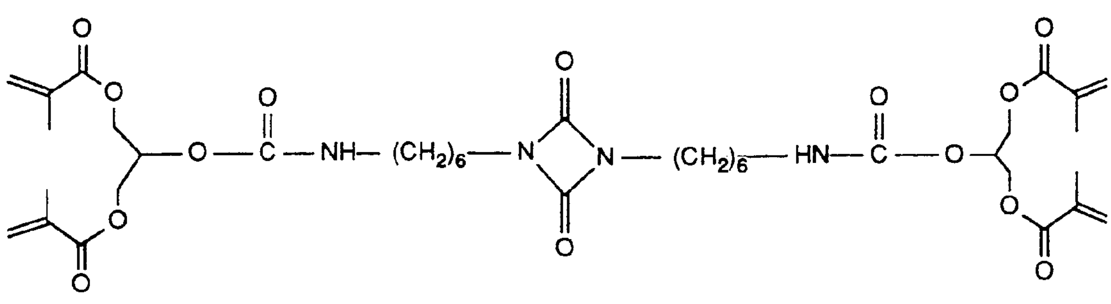

M-4

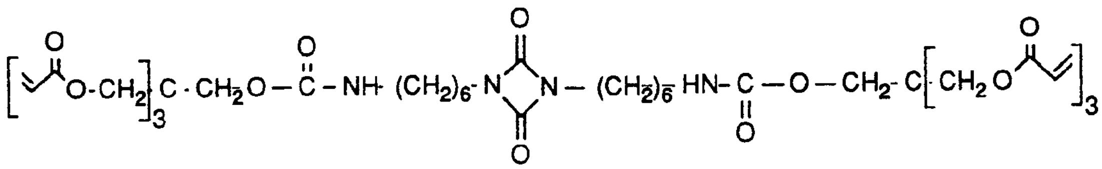

M-5

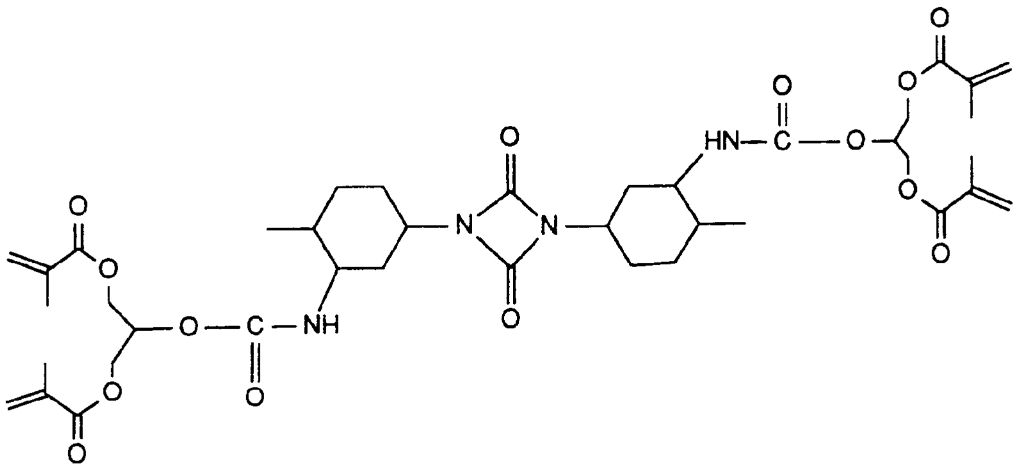

M-6

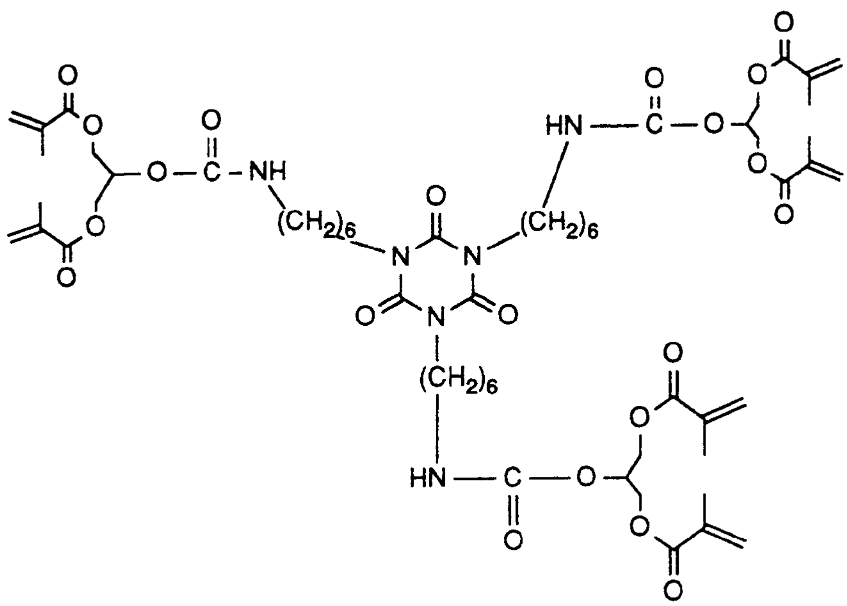

Es wurde gefunden, daß die erfindungsgemäßen multifunktionellen Monomeren sehr gut fotopolymerisierbar sind und bereits bei niedrigen Umsätzen, z.B. in der Größenordnung von 10 %, eine Verfestigung und damit eine deutliche Abnahme der Klebrigkeit und thermischen Übertragbarkeit erleiden und so eine schnelle Differenzierung zwischen belichteten und unbelichteten Teilen erlauben.

Die Synthese der multifunktionellen (Meth)acrylate erfolgt durch Umsetzung von Diisocyanaten oder Triisocyanaten mit Hydroxyalkyl(meth)acrylaten oder N-alkylierten Aminoalkyl(meth)acrylaten entsprechend dem

folgenden Reaktionsschema:

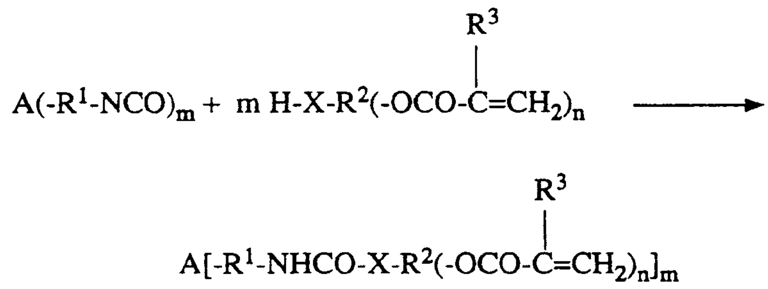

Die Synthese kann bei niedrigviskosen Reaktanten in Substanz durchgeführt werden. In vielen Fällen ist jedoch die Anwendung eines inerten Lösungsmittels vorteilhaft. Im allgemeinen wird ein Katalysator verwendet. Sofern $R^1$ ein aromatischer Rest ist, kann auf die Anwendung des Katalysators verzichtet werden. Die Reaktionstemperatur liegt im Bereich von 0 bis 120°C, vorzugsweise 20 bis 60°C. Der Reaktionsverlauf kann durch die Kontrolle des Isocyanatgehaltes mittels IR-Spektoskopie und/oder Titration verfolgt werden.

Geeignete Lösungsmittel sind z.B. Aceton, Methylethylketon, Chloroform, Tetrahydrofuran, Dioxan, Methylenchlorid, Toluol und Acetonitril.

Im allgemeinen wird die Synthese unter weitgehendem Ausschluß von Wasser durchgeführt. Besonders bevorzugt wird eine maximale Menge an Wasser von weniger als 0,1 Gew.-%, bezogen auf die Gesamtmenge der Reaktanten.

Katalysatoren für die Synthese sind im allgemeinen Metallsalze höherer Fettsäuren. Bevorzugte Katalysatoren können beispielsweise Dibutylzinnlaurat, Dibutylzinnmethoxid und Zinn-(II)-octoat sein. Als Katalysatoren können aber auch Verbindungen mit tertiären Aminogruppen, wie Triethylamin, Pyridin, 2-Methylpyridin, N,N-Dimethylpiperazin und N,N-Dimethylbenzylamin verwendet werden. Es ist auch möglich, Titanverbindungen wie Tetrabutyl-titanat einzusetzen.

Im allgemeinen wird der Katalysator in einer Menge von 0,1 bis 2,5 Gew.-%, bevorzugt 0,1 bis 1,5 Gew.-%, bezogen auf die Gesamtmenge der Reaktanten, eingesetzt werden.

In einer bevorzugten Ausführungsform kann die Synthese in Gegenwart eines Polymerisationsinhibitors durchgeführt werden. Polymerisationsinhibitoren sind an sich bekannt (Ullmanns Enzyklopädie der techn. Chemie, 4. Auflage, Verlag Chemie Weinheim, Band 8, Seiten 19-45). Beispielsweise seien 2,6-Di-tert-butyl-4-methylphenol, Hydrochinon, Hydrochinonmonomethylether genannt.

Es ist auch möglich, als Polymerisationsinhibitor Sauerstoff, z.B. Luftsauerstoff, zu verwenden, der in das Reaktionsgemisch eingeleitet wird.

Im allgemeinen wird der Polymerisationsinhibitor in einer Menge von 0,01 bis 1,0 Gew.-%, bevorzugt von 0,1 bis 0,2 Gew.-%, eingesetzt.

Die fotopolymerisierbare Zusammensetzung wird in bildmäßiger Verteilung einer aktinischen Bestrahlung ausgesetzt, um sie bildmäßig entsprechend der bildmäßigen Verteilung der aktinischen Bestrahlung zu härten. Es kann sich dabei um eine Kontaktbelichtung mit ultravioletter Strahlung, eine Belichtung mittels einer Kamera, eine zeilenweise Belichtung (scanning exposure) oder eine Laserbelichtung handeln. Als Lichtquelle können Tageslicht, Glühlampen, Quecksilberdampflampen, Halogenlampen, Xenonlampen, Fluoreszenzlampen, lichtemittierende Dioden, Laser, Elektronenstrahlen oder Röntgenstrahlen verwendet werden.

Bei einer bevorzugten Ausführungsform der vorliegenden Erfindung sind die Monomeren der allgemeinen Formel I in einer auf einen Schichtträger aufgetragenen thermoplastischen Schicht aus einem thermoplastischen Polymer enthalten, vorzugsweise in einer Menge zwischen 3 und 97 Gew.-%. Nach bildmäßiger Belichtung wird das erfindungsgemäße Bilderzeugungselement (Donorelement) oder ein Akzeptorelement oder beide separat erwärmt, bevor sie miteinander in Kontakt gebracht werden, oder die Erwärmung findet statt, während sich Donorelement und Akzeptorelement miteinander in Kontakt befinden. Nach der Erwärmung und dem bildmäßigen Übertrag können Donorelement und Akzeptorelement voneinander wieder getrennt werden.

Die erwähnte thermoplastische Schicht ist vorzugsweise fest bei Temperaturen unterhalb von 40°C; bei Temperaturen zwischen 40°C und 250°C ist sie in nicht belichteten oder nur unzureichend belichteten Teilen übertragbar.

Geeignete thermoplastische Polymere für die Verwendung in Zusammenhang mit vorliegender Erfindung schließen ein:

(A) Polyester und Polyestergemische aus Alkandiolen, z.B. Polymethylenglykol der Formel $HO-(CH_2)_v-OH$,

wobei v eine ganze Zahl von 2 bis 10 bedeutet, und zwei oder mehr der Dicarbonsäuren Terephthalsäure, Isophthalsäure, Sebacinsäure und Hexahydrophthalsäure;

(B) Nylons oder Polyamide, z.B. N-Methoxymethylpolyhexamethylenadipamid;

(C) Vinylidenchlorid-Copolymere, z.B. Vinyliden/Acrylnitril-, Vinylidenchlorid/Methacrylat- und Vinylidenchlorid/Vinylacetat-Copolymere;

(D) Ethylen/Vinylacetat-Copolymer;

(E) Celluloseether, z.B. Methylcellulose, Ethylcellulose und Benzylcellulose;

(F) Polyethylen;

(G) Synthetische Kautschuke, z.B. Butadien/Acrylnitril-Copolymere und Polymere aus 2-Chlor-1,3-butadien;

(H) Celluloseester, z.B. Celluloseacetat, Celluloseacetatsuccinat, Celluloseacetatbutyrat und Cellulosenitrat;

(I) Polyvinylester, z.B. Polyvinylacetat/Acrylat, Polyvinylacetat/Methacrylat und Polyvinylacetat;

(J) Polyacrylate und Polymethacrylate, z.B. Polymethylmethacrylat;

(K) Hochmolekulare Polyethylenoxide von Polyglykolen mit durchschnittlichem Molekulargewicht von ca. 4000 bis 1 000 000;

(L) Polyvinylchlorid und Copolymere; z.B. Polyvinylchlorid/Acetat, Polyvinylchlorid/Acetat/Alkohol;

(M) Polyvinylacetal, z.B. Polyvinylbutyral oder Polyvinylformal;

(N) Polyformaldehyde;

(O) Polyurethane;

(P) Polycarbonate;

(Q) Polystyrol und dessen Copolymere, z.B. Polystyrol/Acrylnitril oder Polystyrol/Acrylnitril/Butadien.

Der fotopolymerisierbaren Zusammensetzung können außerdem nicht thermoplastische polymere Verbindungen zugesetzt werden, um der Zusammensetzung bestimmte erwünschte Eigenschaften zu verleihen, z.B. zur Verbesserung der Haftung an dem ursprünglichen Träger oder nach dem Übertrag an dem Träger des Akzeptorelements, sowie zur Verbesserung der mechanischen oder chemischen Stabilität. Geeignete, nicht thermoplastische polymere Verbindungen schließen Polyvinylalkohol, Cellulose, wasserfreie Gelatine, Phenolharze und Melaminformaldehydharze ein. Falls gewünscht, können die fotopolymerisierbaren Schichten auch nicht mischbare polymere oder nicht polymere organische oder inorganische Füllstoffe oder Verstärkungsmittel enthalten, die im wesentlichen bei den für die Belichtung verwendeten Wellenlängen transparent sind, z.B. organophile Siliciumverbindungen, Bentonite, Kieselsäure, pulverisiertes Glas, kolloidaler Kohlenstoff, sowie verschiedene Arten von Farbstoffen und Pigmenten in je nach den gewünschten Eigenschaften wechselnden Mengen. Die Füllstoffe sind für die Verbesserung der Festigkeit der Zusammensetzung, die Verminderung der Klebrigkeit und außerdem als Färbemittel geeignet.

Des weiteren können Mittel zur Verbesserung der Benetzbarkeit oder der Haftung der thermoplastischen Schicht zugesetzt werden. Geeignete Mittel sind beispielsweise Silicone, Silicium enthaltende Polymere, z.B. ein Poly(dimethylsiloxan)-polyether-Copolymer, Poly(dimethylsiloxan)-polyester, Silicium enthaltende oberflächenaktive Mittel, Fluor enthaltende Copolymere und Fluor enthaltende Netzmittel.

Die Monomeren entsprechend der allgemeinen Formel I können mit anderen polymerisierbaren ethylenisch ungesättigten Verbindungen, beispielsweise den aus der europäischen Patentanmeldung 91 200 468.6 (05.03.91) bekannten Monomeren, vermischt werden. Weitere geeignete polymerisierbare ethylenisch ungesättigte Verbindungen, die gemäß vorliegender Erfindung verwendet werden können, sind beispielsweise ungesättigte Ester von Polyolen, insbesondere solche von α-Methylencarbonsäuren, z.B. Ethylendiacrylat, Glycerintrimethacrylat, Ethylendimethacrylat, 1,3-Propandioldimethacrylat, 1,2,4-Butantrioltrimethacrylat, 1,4-Cyclohexandioldimethacrylat, Hydrochinondimethacrylat, Pentaerythritoltetramethacrylat, 1,5-Pentandioldimethacrylat, Bisacrylate und Methacrylate von Polyethylenglykolen mit Molekulargewichten von 200 bis 500: ungesättigte Amide, insbesondere solche von gegebenenfalls durch Sauerstoffatome unterbrochenen α,ω-Diaminen wie Methylenbisacrylamid, Methylenbismethacrylamid, 1,6-Hexamethylenbisacrylamid, Diethylentriamintrismethacrylamid, Bis-(γ-methacrylamidopropoxy)-ethan, β-Methacrylamidoethylmethacrylat, N-(β-hydroxyethyl)-β-(methacrylamido)-ethylacrylat und N,N-bis-(β-methacryloyloxyethyl)-acrylamid; Vinylester, z.B. Divinylsuccinat, Divinyladipat, Divinylphthalat, Divinylbutan-1,4-disulfonat; und ungesättigte Aldehyde, z.B. Hexadienal. Andere geeignete polymerisierbare ethylenisch ungesättigte Verbindungen, die gemäß vorliegender Erfindung verwendet werden können, sind Polymere und/oder Oligomere mit zwei oder mehr polymerisierbaren Funktionen z.B. acrylierte Epoxide, Polyesteracrylate, Urethanacrylate.

Die Menge an Monomeren gemäß der allgemeinen Formel I und gegebenenfalls der Comonomeren variiert mit dem im einzelnen ausgewählten thermoplastischen Polymer und/oder anderen verwendeten Zusatzstoffen.

Die fotopolymerisierbare Zusammensetzung gemäß der vorliegenden Erfindung enthält des weiteren wenigstens einen Fotoinitiator. Vorzugsweise verwendete Fotoinitiatoren sind Polymerisationsinitiatoren, die bei Temperaturen unterhalb von 185°C thermisch inaktiv und durch aktinisches Licht aktivierbar sind. Beispiele solcher Initiatoren schließen substituierte und unsubstituierte vielkernige Chinone ein, d.h. Verbindungen mit zwei an Ring-Kohlenstoffatome in einem konjugierten sechsgliedrigen carbocyclischen Ring gebundenen Carbonylgruppen, wobei wenigstens ein aromatischer carbocyclischer Ring an den die Carbonylgruppen tragenden Ring ankondensiert ist. Solche Initiatoren schließen ein 9,10-Anthrachinon, 1-Chloranthrachinon, 2-Chloranthrachinon, 2-Methylanthrachinon, 2-tert.-Butylanthrachinon, Octamethylanthrachinon, 1,4-Naphthochinon, 9,10-Phenanthrenchinon, 1,2-Benzanthrachinon, 2,3-Dichlornaphthochinon, das Natriumsalz von Anthrachinon-α-sulfonsäure, 3-Chlor-2-methylanthrachinon und 1,2,3,4-Tetrahydrobenzo[a]anthracen-7,12-dion. Weiter brauchbare Fotoinitiatoren sind beschrieben in US-PS 2 760 863 und schließen vicinale Ketaldonylverbindungen ein wie Diacetyl, Benzil, α-Ketaldonylalkohole wie Benzoin, Pivalon, Acyloinether wie beispielsweise Benzoinmethyl- und -ethylether; α-kohlenwasserstoff-substituierte aromatische Acyloine einschließlich Methylbenzoin, α-Allylbenzoin und α-Phenylbenzoin. Weitere geeignete Fotoinitiatoren sind beschrieben in "Photoreactive Polymers" von Arnost Reiser, "Organic photochemical imaging systems" von G.A. Delzenne in "UV-Curing Chemistry: Past, Present, and Future" von Christian Decker, veröffentlicht in Journal of Coatings Technology, Vol. 59, Nr. 751, August 1987, Seiten 97-106, sowie in EP-A-O 362 827 und US-A-3 558 309.

Gemäß vorliegender Erfindung können der fotopolymerisierbaren Zusammensetzung auch thermische Polymerisationsinhibitoren zugesetzt werden. Geeignete Inhibitoren sind beispielsweise p-Methoxyphenol, Hydrochinon, alkyl- und acylsubstituierte Hydrochinone und Chinone, tert.-Butylbrenzkatechin, Pyrogallol, Kupferverbindungen, Naphthylamine, β-Naphthol, Kupfer-II-chlorid, 2,6-Bis-tert.-butyl-p-cresol, Fotothiazin, Pyridin, Nitrobenzol und Dinitrobenzol, p-Toluchinon und Chloranil.

Verschiedene Farbstoffe, Pigmente, thermografische Verbindungen, UV-Absorber, Antioxidantien und farbbildende Komponenten können den fotopolymerisierbaren Zusammensetzungen beigefügt werden, um eine Vielzahl von erwünschten Ergebnissen nach dem Wärmeübertrag zu erhalten. Jedoch sollten diese zusätzlichen Stoffe bei der Belichtungswellenlänge möglichst wenig Licht absorbieren und die Polymerisationsreaktion möglichst wenig inhibieren.

Unter den für die Erfindung geeigneten Farbstoffen sind Fuchsine (C.I. 42510), Auramine Base (C.I. 410003), Calcocid Green S (C.I. 44000), Para Magenta (C.I. 42500), Tryparosan (C.I. 42505), New Magenta (C.I. 42520), Acid Violet RRL (C.I. 42425), Red Violet 5RS (C.I. 42960), C.I. Solvent Blue 36 (C.I. 61551), Nile Blue 2B (C.I. 51185), New Methylene Blue GG (C.I. 51195), C.I. Basic Blue 20 (C.I. 42585, Iodine Green (C.I. 42556), Night Green B (C.I. 42415), C.I. Direct Yellow 9 (C.I. 19540), C.I. Acid Yellow 17 (C.I. 18965), C.I. Acid Yellow 29 (C.I. 18900), Tartrazine (C.I. 19140), Supramine Yellow G (C.I. 19300), Buffalo Black 10B (C.I. 27790), Naphtalene Black 12R (C.I. 20350), Fast Black L (C.I. 51215), und Ethyl Violet (C.I. 42600).

Geeignete Pigmente schließen beispielsweise ein: $TiO_2$, kolloidalen Kohlenstoff, Graphit, Phosphorteilchen, keramische Teilchen, Tonteilchen, Metallpulver wie Aluminium, Kupfer, magnetisches Eisen und Bronze. Die Pigmente sind besonders brauchbar, wenn sie in die fotoempfindliche Schicht oder in eine benachbarte nicht lichtempfindliche Schicht eingelagert werden, z.B. in eine Antihalo-Schicht oder eine Schicht zur Verbesserung der Haftung zwischen dem Träger und der fotoempfindlichen Schicht.

Brauchbare thermografische Zusätze, z.B. 3-Cyan-4,5-dimethyl-5-hydroxy-3-pyrrolin-2-on, und Aktivatoren, z.B. Kupferacetat, sind beschrieben in den folgenden US-Patenten: 2 825 494, 2 637 657, 2 665 654, 2 663 655, 2 663 656 und 2 663 657.

Geeignete farbbildende Komponenten, die bei Einwirkung von Warme oder wenn sie mit anderen farbbildenden Komponenten in einem Akzeptorelement in Kontakt gebracht werden, farbige Verbindungen bilden, sind beispielsweise:

(1) Organische und anorganische Komponenten: Dimethylglyoxim und Nickelsalze; Phenolphthalein und Natriumhydroxid; Stärke/Kaliumiodid und oxidierende Mittel, z.B. Peroxide, Phenole und Eisensalze; Thioacetamid und Bleiacetat, Silbersalze und Reduktionsmittel, z.B. Hydrochinon.

(2) Anorganische Komponenten: Eisen-III-Salze und Kaliumthiocyanat; Eisen-II-Salze und Kaliumferricyanid; Kupfer oder Silbersalze und Sulfidionen; Bleiacetat und Natriumsulfid.

(3) Organische Komponenten: 2,4-Dinitrophenylhydrazin und Aldehyde oder Ketone; Diazoniumsalz und Phenol oder Naphthol, z.B. Benzoldiazoniumchlorid und β-Naphthol; p-Dimethylaminobenzaldehyd und p-Diethylaminoanilin.

Das Bilderzeugungselement der vorliegenden Erfindung kann weitere Schichten enthalten, z.B. eine Schicht zur Verbesserung der Haftung der fotopolymerisierbaren Schicht am Schichtträger oder eine Strippingschicht. Besonders vorteilhaft ist es, zwischen dem Schichtträger und der lichtempfindlichen fotopolymerisierbaren Schicht, an letztere unmittelbar angrenzend, eine funktionelle Zwischenschicht anzuordnen,

die beispielsweise ein Polymer mit polymerisierbaren ethylenisch ungesättigten Gruppen enthält. Eine solche funktionelle Zwischenschicht ist beispielsweise in EP-A-0522616 beschrieben.

Als Schichtträger für das erfindungsgemäße Bilderzeugungselement sind Träger geeignet, die bei den für die Übertragung der nicht oder nicht ausreichend belichteten Teile der fotopolymerisierbaren Zusammensetzung auf das Akzeptormaterial erforderlichen Temperaturen stabil sind. Beispiele geeigneter Träger sind solche aus Polyester, z.B. Polyethylenterephthalat, Glas, Holz, Papier, Polyethylenbeschichtetes Papier, Celluloseester, z.B. Celluloseacetat, Cellulosepropionat, Cellulosebutyrat, Polycarbonat, Polyvinylchlorid, Polyimid, Polypropylen.

Ebenso muß auch das Akzeptormaterial, auf das das Bild übertragen wird, bei den Verarbeitungstemperaturen stabil sein. Das im Einzelfall verwendete Akzeptormaterial richtet sich nach der Haftung des Bildes auf dem Bilderzeugungselement. Geeignete Akzeptormaterialien schließen Papier, Pappe, Metallfolien und -gewebe, z.B. aus Aluminium, Kupfer, Eisen, Bronze, Polyethylen, Polyester, z.B. Polyethylenterephthalat, opak aufgeschäumte oder pigmentierte Polyester, Celluloseester, Seide, Baumwolle, Polycarbonat, Polyvinylchlorid, Polypropylen und polyethylenkaschiertes Papier, ein.

Das Akzeptorelement kann eine hydrophile Oberfläche aufweisen oder eine Schicht mit bevorzugter Haftung für die nicht belichteten Teile der Schicht der fotopolymerisierbaren Zusammensetzung, oder es kann auf seiner Oberfläche Verbindungen enthalten, die mit übertragenen Verbindungen reagieren, um einen Unterschied in Farbe, Hydrophilie, Leitfähigkeit und dgl. auf der Oberfläche des Akzeptormaterials zu erzeugen.

Bilderzeugungselement und Akzeptormaterial können auch vor der Belichtung in Kontakt gebracht werden oder als einheitliches Element vorliegen. Ein solches Element wird als Monosheet-Material bezeichnet und setzt voraus, daß entweder Vorder- oder Rückseite transparent sind für die zur Belichtung der fotopolymerisierbaren Zusammensetzung verwendete Strahlung.

In einer besonders bevorzugten Ausführungsform vorliegenden Erfindung enthält das Akzeptormaterial eine hydrophile Oberfläche. Die Übertragung der nicht oder unzureichend belichteten Teile der fotopolymerisierbaren Zusammensetzung auf ein Akzeptorelement mit hydrophiler Oberfläche hat eine bildmäßige Differenzierung zwischen hydrophilen und hydrophoben Teilen zur Folge, die für ein Druckverfahren mit öliger oder fettiger Tinte ausgenutzt werden kann. Die hydrophoben Teile sind zur Aufnahme von lithografischer Tinte bereit während die hydrophilen Teile, besonders nach Befeuchtung mit Wasser, keine Tinte annehmen. Die mit Tinte eingefärbten Teile stellen das Druckbild dar und die Tinte abweisenden Bereiche bilden den Bildhintergrund.

Nach der Übertragung der nicht oder unzureichend belichteten Teile der fotopolymerisierbaren Zusammensetzung kann es vorteilhaft sein, das übertragene Bild einer gleichmäßigen Belichtung oder Erwärmung auszusetzen, um die Stabilität zu verbessern. Eine solche Maßnahme empfiehlt sich besonders dann, wenn das übertragene Bild als Druckvorlage verwendet werden soll.

Akzeptormaterialien mit hydrophiler Oberfläche sind beispielsweise Metallträger wie Aluminium oder Zink, Polyesterfilm oder Papierunterlagen. Diese Träger können, falls sie nicht selbst ausreichend hydrophil sind, zuerst mit einer hydrophilen Schicht beschichtet werden. Eine beosnders geeignete hydrophile Schicht ist eine Schicht aus Polyvinylalkohol, die mit einem Tetraorthosilikat, z.B. einem $TiO_2$ enthaltenden Tetramethylorthosilikat oder Tetraethylorthosilikat, gehärtet wurde wie beispielsweise beschrieben in US-A-3 971 660.

Ein besonders geeigneter Metallträger ist Aluminium. Geeignete Aluminiumträger für die Verwendung gemäß vorliegender Erfindung sind Aluminiumfolien aus Reinaluminium oder eine Aluminiumlegierung, deren Aluminiumgehalt wenigstens 95 % beträgt. Eine geeignete Legierung enthält beispielsweise 99,55 Gew.-% Aluminium, 0,29 Gew.-% Eisen, 0,10 Gew.-% Silizium, 0,004 Gew.-% Kupfer, 0,002 Gew.-% Mangan, 0,02 Gew.-% Titan und 0,03 Gew.-% Zink. Die Dicke einer solchen Folie liegt üblicherweise zwischen 0,13 und 0,5 mm.

Die Herstellung einer für den lithografischen Offsetdruck geeigneten Aluminium- oder Aluminiumlegierung-Folie umfaßt folgende Schritte: Aufrauhung, Anodisierung und gegebenenfalls Versiegelung.

Aufrauhung und Anodisierung der Folie sind notwendig, um gemäß vorliegender Erfindung hochqualitative Drucke zu ermöglichen. Versiegelung ist nicht notwendig, aber kann die Druckergebnisse weiter verbessern.

Aufrauhung der Aluminiumoberfläche kann mechanisch oder elektrolytisch in bekannter Weise durchgeführt werden. Die durch die Aufrauhung erreichte Rauheit wird in µm als Abweichung von einer Mittellinie gemessen und beträgt vorzugsweise ca. 0,2 bis 1,5 µm.

Die Anodisierung der Aluminiumfolie kann durchgeführt werden in elektrolytischen Bädern, z.B. Chromsäure. Oxalsäure, Natriumcarbonat, Natriumhydroxid und deren Mischungen. Bevorzugt wird die Anodisierung von Aluminium und verdünnter wäßriger Schwefelsäure durchgeführt bis die erwünschte Dicke der Anodisierungsschicht erreicht ist. Die Aluminiumfolie kann auf beiden Seiten anodisiert werden. Die Dicke der Anodisierungsschicht wird genau bestimmt an einem Mikroschnitt, aber sie kann ebenso ermittelt werden durch Auflösung der Anodisierungsschicht und Wiegen der Folie vor und nach der Auflösungsbehandlung. Gute Ergebnisse werden erhalten mit einer Anodisierungsschicht, die ungefähr von 0,4 bis ungefähr 2,0 μm beträgt. Zur Verbesserung der Bildschärfe und dementsprechend der Schärfe der Druckkopie kann die Anodisierungsschicht in der Masse eingefärbt sein mit einem Antihalo-Farbstoff oder Pigment wie beispielsweise beschrieben in JP-A-58-14797.

Nach der Anodisierungsbehandlung kann die anodische Oberfläche versiegelt werden. Versiegelung der Poren der bei der Anodisierung gebildeten Aluminiumoxidschicht ist eine bekannte Technik, die beispielsweise beschrieben ist in der "Belgisch-Nederlands tijdschrift voor Oppervlaktetechnieken van materialen", 24. Jahrgang/Januar 1980. Man kennt verschiedene Arten der Versiegelung von porösen, anodisierten Aluminiumoberflächen. Eine vorteilhafte Methode ist die Hydratationsversiegelung, bei der die Poren ganz oder teilweise durch Wasseraufnahme geschlossen werden, so daß sich hydratisierte nadelförmige Aluminiumoxidkristalle (Böhmit) bilden. Die anodisierte Oberfläche der Aluminiumfolie kann hierzu mit Wasser bei 70 bis 100°C oder mit Dampf behandelt werden. Das Wasser kann hierzu Zusätze enthalten, z.B. Nickelsalze, um den Versiegelungseffekt zu verbessern. Die Versiegelung kann auch durchgeführt werden durch Behandlung der anodischen Oberfläche mit einer wäßrigen Lösung von Phosphationen oder Silikaten. Aufgrund der Versiegelungsbehandlung wird die anodische Schicht im wesentlich nicht porös gemacht, so daß sie für die Herstellung einer größeren Anzahl von Drucken geeignet sind. Als Ergebnis der Versiegelungsbehandlung kann das Auftreten von Schleier in den Nichtbild-Teilen der Druckplatte weitgehend vermieden werden.

Die Aufrauhung, die Anodisierung und die Versiegelungsbehandlung der Aluminiumfolie kann durchgeführt werden wie beispielsweise beschrieben in US-A-3 861 917.

Das Bilderzeugungselement vorliegender Erfindung eignet sich in gleicher Weise auch für die Herstellung einer elektrostatischen Druckvorlage. Hierbei wird die bildmäßig belichtete fotopolymerisierbare Zusammensetzung gemäß vorliegender Erfindung auf ein Akzeptorelement übertragen, das eine leitfähige Oberfläche enthält oder eine Oberfläche mit Verbindungen, die mit übertragenen Verbindungen reagieren, so daß eine bildmäßige Differenzierung in der elektrischen Leitfähigkeit erhalten wird. Vorzugsweise wird ein Akzeptormaterial mit einer leitfähigen Oberfläche verwendet, z.B. ein mit einer metallischen Schicht von beispielsweise Aluminium versehener Polyethylenterephthalat-Träger. Nach der Übertragung der bildmäßig belichteten fotopolymerisierbaren Zusammensetzung auf ein solches Material bleiben die Teile, an denen kein Übertrag stattgefunden hat und die den belichteten Teilen der fotopolymerisierbaren Zusammensetzung in dem Bilderzeugungselement entsprechen, leitfähig, während die anderen Teile nichtleitend geworden sind. Auf diese Weise wird eine elektrostatische Druckvorlage erhalten.

Das Akzeptormaterial kann ein Träger sein, der mit einer Metallschicht, beispielsweise Kupfer, versehen ist. Eine gedruckte Schaltung kann auf diese Weise hergestellt werden in einer nachfolgenden Ätzbehandlung, bei der die bildmäßig übertragene fotopolymerisierbare Zusammensetzung als Fotoresist wirkt.

Nach einer anderen Ausführungsform der vorliegenden Erfindung können Farbbilder hergestellt werden. Hierzu werden mindestens drei Bilderzeugungselemente, die einen Gelbfarbstoff, einen Magentafarbstoff und einen Cyanfarbstoff bzw. entsprechende Farbstoffpigmente in oder unterhalb der fotopolymerisierbaren Zusammensetzung enthalten, bildmäßig belichtet mit einem Blau-, Grün- oder Rot-Farbauszug der Vorlage. Gegebenenfalls kann auch ein viertes Bilderzeugungselement verwendet werden, das einen schwarzen Farbstoff oder Farbstoffpigment enthält. Diese Bilderzeugungselemente werden nacheinander in Kontakt mit dem gleichen Akzeptorelement, z.B. einem Papier, erhitzt, um den Übertrag der einzelnen unterschiedlichen Farbauszüge zu bewirken. Es liegt auf der Hand, daß der Übertrag der unterschiedlichen Farbauszüge des Bildes paßgenau erfolgen muß, um eine farbgetreue Reproduktion des ursprünglichen Bildes zu erhalten.

Nach einer weiteren Ausführungsform des erfindungsgemäßen Verfahrens können Bilder hergestellt werden unter Verwendung eines Monosheet-Materials, das auf einen transparenten Schichtträger in der folgenden Reihenfolge eine lichtempfindliche Schicht mit dem erfindungsgemäßen Monomer, eine Pigmentschicht und eine selbsttragende oder auf einen zweiten Schichtträger angeordnete Akzeptorschicht enthält. Nach bildmäßiger Belichtung durch den transparenten Schichtträger dringen bei der nachfolgenden Entwicklung die nicht oder nur unzureichend gehärteten Teile der lichtempfindlichen Schicht in die Pigmentschicht ein und werden zusammen mit dem Pigment auf die Akzeptorschicht übertragen. Letztere besteht beispielsweise aus Papier oder Polyesterfilm.

Der Ausdruck "übertragen" ist dabei so zu verstehen, daß die nicht oder unzureichend gehärteten Teile der lichtempfindlichen Schicht zusammen mit dem Pigment an der Akzeptorschicht haften bleiben, wenn diese nach der thermischen Übertragung von dem Bilderzeugungselement abgezogen wird. Bei dieserAusführungsform sollte die Pigmentschicht hinreichend porös sein, um das Eindringen der nicht oder unzureichend gehärteten Teile der lichtempfindlichen Schicht zu ermöglichen. Aus diesem Grund wird die Menge an Bindemittel in der Pigmentschicht möglichst gering gehalten. Bindemittelfreie Pigmentschichten sind bevorzugt. Als Pigmente in dieser Ausführungsform eignen sich die zuvor erwähnten Pigmente.

Ein Bild kann auch dadurch hergestellt werden, daß ein bildmäßig belichtetes Bilderzeugungselement, das die fotopolymerisierbare Zusammensetzung gemäß vorliegender Erfindung als äußere Schicht enthält, auf eine Temperatur erhitzt wird, die ausreicht, um die nicht oder unzureichend belichteten Teile klebrig zu machen während die belichteten Teile aufgrund der Fotopolymerisation nicht klebrig werden. Bei nachfolgender Behandlung mit einem Puder aus einem Farbstoffpigment, z.B. Ruß, kann das Bild entwickelt werden, da das Pigment an den klebrigen Teilen des erhitzten Bilderzeugungselementes haftet. Nach Abkühlung des mit Farbpuder versehenen Bilderzeugungselementes auf Raumtemperatur kann das Bilderzeugungselement gleichmäßig belichtet werden, um auch die bepuderten Teile zu härten. Hierbei wird ein Bild hoher Qualität und hohen Kontrastes erhalten.

Die oben beschriebenen Ausführungsformen der vorliegenden Erfindung sind wegen der dabei verwendeten trockenen Entwicklung leicht handhabbar und umweltfreundlich und deswegen bevorzugt. Für den Fachmann ist jedoch klar, daß die bildmäßig belichtete fotopolymerisierbare Zusammensetzung ebenso unter Verwendung von Lösungsmitteln entwickelt werden kann, um die nicht oder unzureichend belichteten Teile aufzulösen. Geeignete Lösungsmittel für die Verwendung in einem solchen Entwicklungsverfahren sind organische Lösungsmittel, z.B. Chloroform, Dichlorethan, Dichlormethan, Toluol, Benzol.

<u>Beispiel 1</u> (Herstellung der Monomeren)

Allgemeine Arbeitsvorschrift

Der NCO- bzw. OH-Gruppengehalt der eingesetzten Isocyanate und Hydroxyalkyl(meth)acrylate wurden quantitativ durch Titration bestimmt, so daß die reagierenden Gruppen in exakt äquimolarer Menge eingesetzt wurden.

Bei Raumtemperatur wurde das entsprechende Isocyanat (0,300 mol NCO) in 250 ml Chloroform vorgelegt und mit 600 ppm Sn-II-isooctonoat (Desmorapid® SO) und 1000 ppm 2,6-Di-tert.-butylkresol (Jonol) versetzt. Zu dieser gerührten Vorlage wurde bei Raumtemperatur über 20 min das Hydroxyalkyl(meth)acrylat zugetropft, wobei die leicht exotherme Reaktion einen Temperaturanstieg von etwa 5°C bewirkte. Es wurde 30 min nachgerührt, auf 60°C erwärmt und bei dieser Temperatur so lange belassen, bis nach Maßgabe der IR-Kontrolle alle Isocyanat-Gruppen vollständig abreagiert waren (zumeist mußte für den vollständigen Umsatz über Nacht gerührt werden, so daß die Reaktion erst am darauffolgenden Morgen abgestellt werden konnte).

Verbindung M-1 aus Tabelle 1

Entsprechend der allgemeinen Vorschrift wurden 0,150 mol Uretdion des Hexamethylendiisocyanats mit 0,300 mol 2-Hydroxyethylacrylat umgesetzt.

Verbindung M-2 aus Tabelle 1

Entsprechend der allgemeinen Vorschrift wurden 0,150 mol Uretdion des Hexamethylendiisocyanats mit 0,300 mol 2-Hydroxyethylmethacrylat umgesetzt.

Verbindung M-3 aus Tabelle 1

Entsprechend der allgemeinen Vorschrift wurden 0,150 mol Uretdion des Hexamethylendiisocyanats mit 0,300 mol Glycerindimethacrylat umgesetzt.

Verbindung M-4 aus Tabelle 1

Entsprechend der allgemeinen Vorschrift wurden 0,150 mol Uretdion des Hexamethylendiisocyanats mit 0,300 mol Pentaerythrittriacrylat umgesetzt.

Verbindung M-5 aus Tabelle 1

Entsprechend der allgemeinen Vorschrift wurden 0,150 mol Uretdion des 1,3-Diisocyanato-4-methyl-cyclohexans mit 0,300 mol Glycerindimethacrylat umgesetzt.

Verbindung M-6 aus Tabelle 1

Entsprechend der allgemeinen Vorschrift wurden 0,100 mol Isocyanurat des Hexamethylendiisocyanats (Desmodur® N 3300 der Bayer AG) mit 0,300 mol Glycerindimethacrylat umgesetzt.

Beispiel 2

Ein Bilderzeugungselement wurde wie folgt hergestellt.

Auf einen mit einer Haftschicht versehenen Schichtträger aus Polyethylenterephthalat mit einer Dicke von 100 µm wurde aufgetragen (pro m²):

a) eine funktionelle Zwischenschicht (1 g) aus Polyvinylalkohol mit 25 mol-% durch Umsetzung mit Methacryldichlorid modifizierten Hydroxylgruppen;

b) eine fotoempfindliche Schicht (3 g), vergossen als Lösung in Methylethylketon aus:

| | |
|---|---|
| 4 Gew.-% | Monomer M-3 |
| 0,66 Gew.-% | Blaufarbstoff (C.I.61551) |
| 0,02 Gew.-% | TEGOGLIDE 410 (Polysiloxan-polyether- Copolymer der Fa. Goldschmidt AG, Essen) |
| 3,5 Gew.-% | Bisimidazol |
| 0,1 Gew.-% | Michlers Keton |
| 0,2 Gew.-% | Mercaptobenzoxazol |

Ein Akzeptorelement wurde wie folgt hergestellt:

Zu 418 g einer Dispersion von 21,5 Gew.-% $TiO_2$ (durchschnittliche Teilchengröße 0,3 bis 0,5 µm) und 2,5 Gew.-% Polyvinylalkohol in entionisiertem Wasser wurden unter Rühren nacheinander zugefügt:

| | |
|---|---|
| 220 g | einer 5 %igen Lösung aus Polyvinylalkohol in Wasser |
| 95 g | einer hydrolysierten 22 %igen Emulsion von Tetramethylorthosilikat in Wasser |
| 22 g | einer 10 %igen Lösung eines Netzmittels. |

Zu dieser Mischung wurde anschließend 245 ml entionisiertes Wasser zugefügt; und der pH wurde auf 4 eingestellt. Diese Dispersion wurde auf einen mit hydrophiler Haftschicht versehenen Schichtträger zu einer Naßschichtdicke von 55 g vergossen und bei 30°C getrocknet.

Das Bilderzeugungselement wurde mit einer transparenten Testvorlage mit einem Strichmuster von 150 Linien pro Zoll in Kontakt gebracht und durch die Restvorlage mit UV-Licht belichtet.

Das belichtete Bilderzeugungselement wurde dann in beschichtungsseitigem Kontakt mit dem hydrophilen Akzeptorelement bei 165°C und mit einer Geschwindigkeit von 1,02 m/min durch eine Rollenlaminatorvorrichtung geführt Anschließend wurden die beiden Elemente getrennt.

Die Probe zeigte eine gute Punktwiedergabe. Mit dem auf dem Akzeptorelement erhaltenen Bild konnte in einer konventionellen Offset-Druckvorrichtung mit herkömmlicher Tinte und anschließender Spülung gedruckt werden. Es wurden gute Kopien erhalten.

Beispiel 3

Wie in Beispiel 2 wurde ein Bilderzeugungselement hergestellt mit dem Unterschied, daß das Monomer M-6 anstelle von Monomer M-3 verwendet wurde. Belichtung und Verarbeitung erfolgte wie in Beispiel 2. Eine gute Punktreproduktion wurde erhalten.

Die Probe zeigte eine gute Punktwiedergabe. Mit dem auf dem Akzeptorelement erhaltenen Bild konnte in einer konventionellen Offset-Druckvorrichtung mit herkömmlicher Tinte und anschließender Spülung gedruckt werden. Es wurden gute Kopien erhalten.

Beispiel 4

Wie in Beispiel 2 beschrieben wurde ein Bilderzeugungselement hergestellt mit dem Unterschied, daß die funktionelle Zwischenschicht einen Auftrag von 2 g und die fotopolymerisierbare Schicht einen Auftrag von 1 g aufwies, und daß über der fotopolymerisierbaren Schicht eine Pigmentschicht mit 5 Gew.-% Ruß und einer Trockenschichtdicke von 1 g angeordnet war.

Das erhaltene Bilderzeugungselement wurde bildmäßig belichtet wie in Beispiel 2 beschrieben und anschließend in Kontakt mit einem Polyethylenterephthalatträger als Bildakzeptormaterial bei 180°C und mit einer Geschwindigkeit von 0,36 m/min durch eine Rollenlaminierungsvorrichtung geführt. Darauf wurden die Elemente auseinandergezogen. Ein Bild mit guter Auflösung wurde erhalten.

Beispiel 5

Ein Bilderzeugungselement wurde hergestellt wie in Beispiel 4 beschrieben, wobei jedoch anstelle des Monomers M-3 das Monomer M-6 verwendet wurde. Belichtung und Verarbeitung erfolgten wie in Beispiel 4 angegeben. Eine gute Punktwiedergabe wurde erhalten.

## Patentansprüche

1. Bilderzeugungselement mit einer auf einen Schichtträger aufgetragenen Schicht einer fotopolymerisierbaren Zusammensetzung, gekennzeichnet durch einen fotopolymerisierbaren Gehalt an mindestens einem Monomer der folgenden Formel

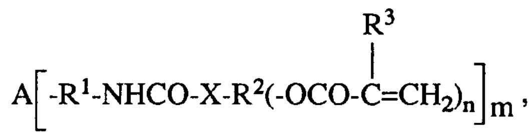

worin bedeuten

A einen zwei- oder dreibindigen kondensierten Harnstoffrest einer der Formeln:

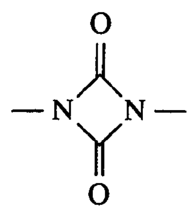 und 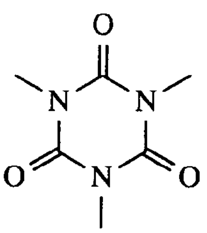 ;

X -O- oder -NR- mit R = $C_1$-$C_{12}$-Alkyl;
$R^1$ einen zweibindigen Kohlenwasserstoffrest mit 2 bis 25 C-Atomen;
$R^2$ einen (n + 1)-bindigen, linearen oder verzweigten, gegebenenfalls durch bis zu 3 O-Atome unterbrochenen Kohlenwasserstoffrest mit 2 bis 18 C-Atomen;
$R^3$ H oder Methyl;
n eine ganze Zahl von 2 bis 5; und
m 2 oder 3.

2. Bilderzeugungselement nach Anspruch 1, dadurch gekennzeichnet, daß die fotopolymerisierbare Zusammensetzung 3 bis 97 Gew.-% eines oder mehrerer Monomerer der Formel I und 97 bis 3 Gew.-% eines thermoplastischen Polymers enthält.

3. Bilderzeugungselement nach einem der Ansprüche 1 und 2, dadurch gekennzeichnet, daß die fotopolymerisierbare Zusammensetzung einen Farbstoff oder ein Farbpigment enthält.

4. Bilderzeugungselement nach einem der Ansprüche 1 bis 3, dadurch gekennzeichnet, daß zwischen dem Schichtträger und der fotopolymerisierbaren Schicht, an letztere angrenzend, eine funktionelle Zwischenschicht angeordnet ist, die ein Polymer mit polymerisierbaren ethylenisch ungesättigten Gruppen enthält.

5. Bilderzeugungselement nach einem der Ansprüche 1 bis 4, dadurch gekennzeichnet, daß über der fotopolymerisierbaren Schicht eine Akzeptorschicht angeordnet ist (Monosheet-Material).

6. Bilderzeugungselement nach Anspruch 5, dadurch gekennzeichnet, daß zwischen der fotopolymerisierbaren Schicht und der Akzeptorschicht eine Pigmentschicht angeordnet ist.

7. Bilderzeugungsverfahren, bei dem ein Bilderzeugungselement nach einem der Ansprüche 1 bis 6 bildmäßig einer aktinischen Belichtung ausgesetzt wird, wodurch die bildmäßig belichteten Teile der fotopolymerisierbaren Schicht gehärtet werden, und bei dem die nicht oder unzureichend gehärteten Teile der

fotopolymerisierbaren Schicht durch Erwärmen auf eine Akzeptorschicht übertragen werden.

**8.** Verbindung der Formel I

$$A\left[-R^1\text{-NHCO-X-}R^2(\text{-OCO-}\overset{R^3}{\overset{|}{C}}=CH_2)_n\right]_m \qquad (I),$$

worin bedeuten

A

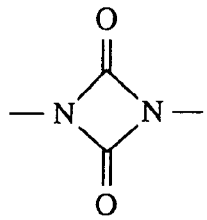

(m = 2)

X -O-;

$R^1$ einen aliphatischen oder cycloaliphatischen Rest mit 5 bis 8 C-Atomen;

$R^2$ einen drei- oder vierbindigen Kohlenwasserstoffrest (n = 2 oder 3) mit 3 bis 6 C-Atomen;

$R^3$ H oder Methyl.

**9.** Verbindung einer der folgenden Formeln

M-3

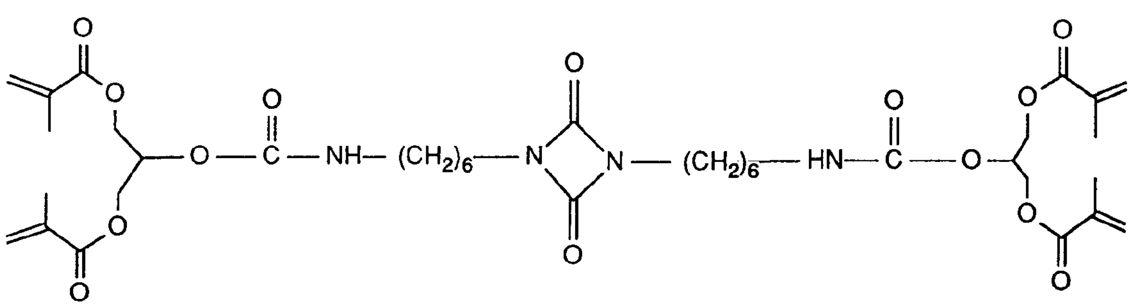

M-4

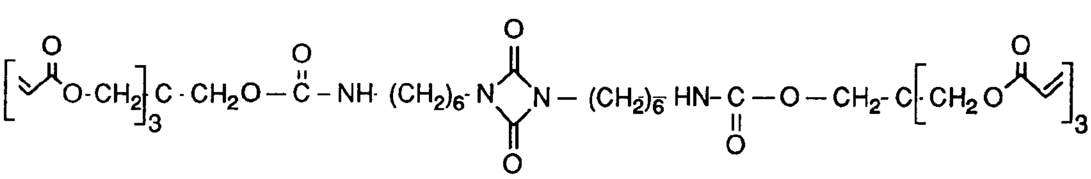

M-5

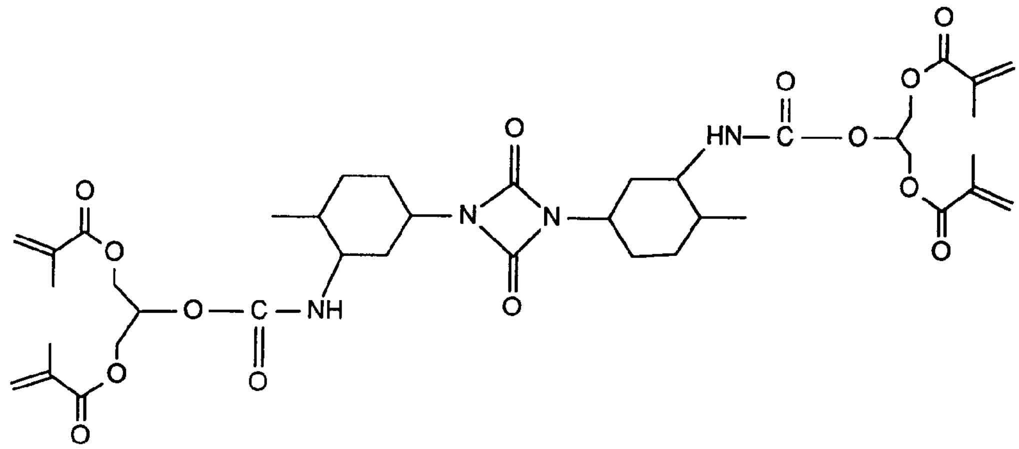

## Claims

1. Imaging element having a layer of a photopolymerisable composition applied to a support, characterised by a photopolymerisable content of at least one monomer of the following formula (I)

$$A[-R^1-NHCO-X-R^2(-OCO-\underset{}{\overset{R^3}{\overset{|}{C}}}=CH_2)_n]_m$$

in which

A means a di- or trivalent condensed urea residue of one of the formulae:

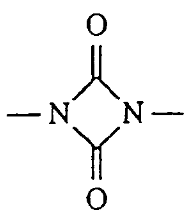 and 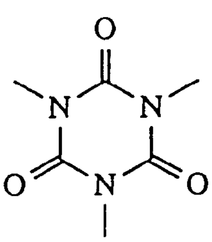 ;

X means -O- or -NR- with R meaning $C_1$-$C_{12}$ alkyl;
$R^1$ means a divalent hydrocarbon residue with 2 to 25 C atoms;
$R^2$ means an (n + 1)-valent, linear or branched hydrocarbon residue with 2 to 18 C atoms, optionally interrupted by up to three O atoms;
$R^3$ means H or methyl;
n means an integer from 2 to 5; and
m means 2 or 3.

2. Imaging element according to claim 1, characterised in that the photopolymerisable composition contains 3 to 97 wt.% of one or more monomers of the formula I and 97 to 3 wt.% of a thermoplastic polymer.

3. Imaging element according to one of claims 1 and 2, characterised in that the photopolymerisable composition contains a dye or colouring pigment.

4. Imaging element according to one of claims 1 to 3, characterised in that a functional interlayer containing a polymer with polymerisable ethylenically unsaturated groups is arranged between the support and the photopolymerisable layer in a position adjacent to the latter.

5. Imaging element according to one of claims 1 to 4, characterised in that an acceptor layer is arranged above the photopolymerisable layer (monosheet material).

6. Imaging element according to claim 5, characterised in that a pigment layer is arranged between the photopolymerisable layer and the acceptor layer.

7. Imaging process in which an imaging element according to one of claims 1 to 6 is exposed with an image using actinic light, so curing the portions of the photopolymerisable layer exposed with the image, and in which the uncured or inadequately cured portions of the photopolymerisable layer are transferred onto an acceptor layer by heating.

8. Compound of the formula I

$$A[-R^1\text{-NHCO-X-}R^2(\text{-OCO-}\overset{\displaystyle R^3}{\overset{|}{C}}\text{=}CH_2)_n]_m \qquad (I),$$

in which

A means

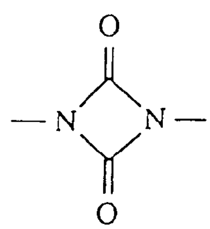

(m = 2)

X means -O-;

$R^1$ means an aliphatic or cycloaliphatic residue with 5 to 8 C atoms;

$R^2$ means a di- or trivalent hydrocarbon residue (n = 2 or 3) with 3 to 6 C atoms;

$R^3$ means H or methyl.

9. Compound of one of the following formulae

M-3

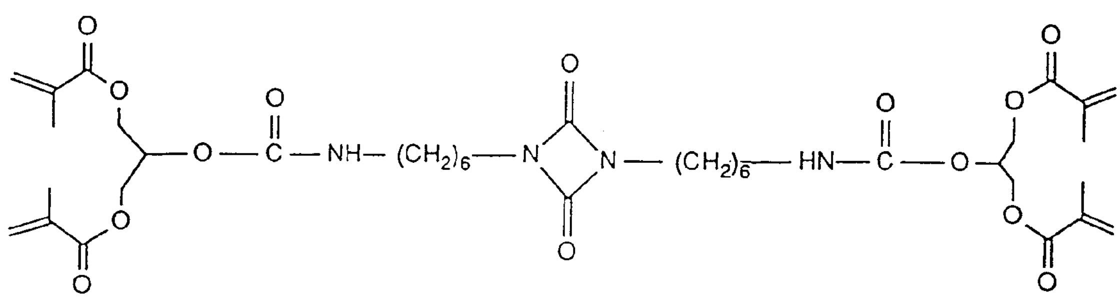

M-4

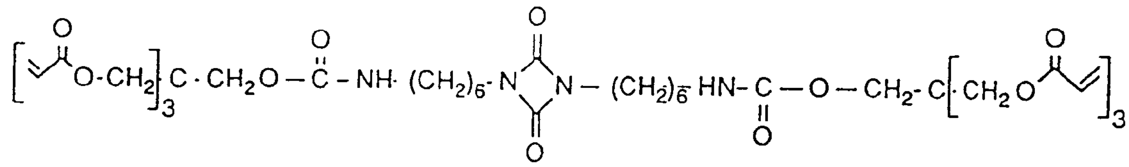

M-5

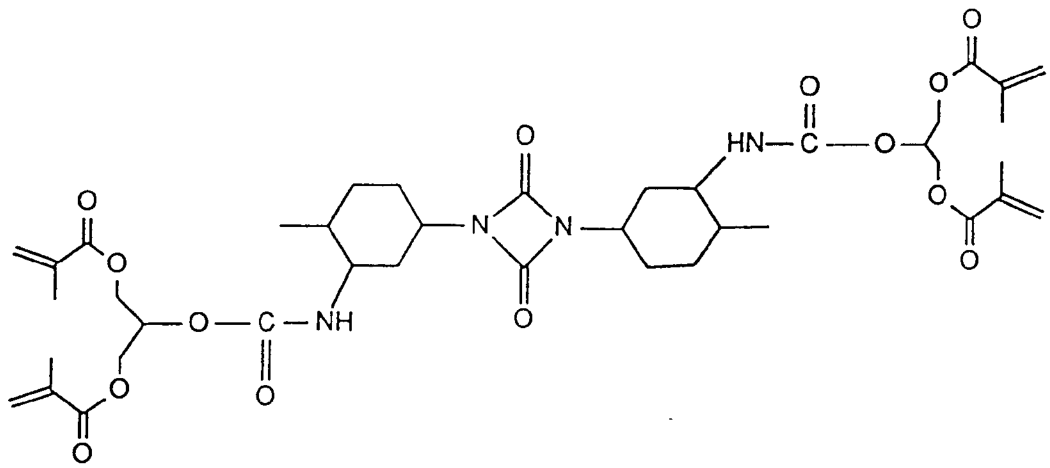

## Revendications

1. Elément de formation d'images avec une couche d'une composition photopolymérisable appliquée sur un support de couches caractérisé par une teneur photopolymérisable d'au moins un monomère de formule suivante:

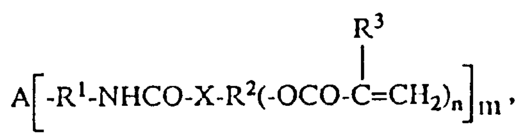

dans laquelle

A désigne un radical urée condensé à deux ou trois liaisons d'une des formules

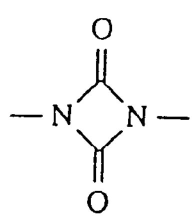 et 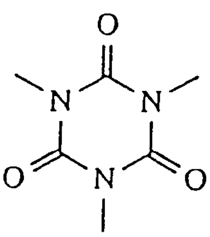 ;

X désigne -O- ou -NR-, R étant un radical alkyle $C_1$-$C_{12}$;

$R^1$ désigne un radical hydrocarbure à 2 liaisons avec 2 à 25 atomes C;

$R^2$ désigne un radical hydrocarbure à (n + 1) liaisons, linéaire ou ramifié, éventuellement interrompu par jusqu'à 3 atomes O et avec 2 à 18 atomes C;

$R^3$ désigne H ou un radical méthyle;
n désigne un nombre entier de 2 à 5; et
m désigne 2 ou 3.

2. Elément de formation d'images selon la revendication 1, caractérisé en ce que la composition photopolymérisable contient 3 à 97% en poids d'un ou plusieurs monomères de formule I et 97% à 3% en poids d'un polymère thermoplastique.

3. Elément de formation d'images selon l'une des revendications 1 et 2, caractérisé en ce que la composition photopolymérisable contient un colorant ou un pigment colorant.

4. Elément de formation d'images selon l'une des revendications 1 à 3, caractérisé en ce qu'entre le porteur de couches et la couche photopolymérisable, adjacente à cette dernière, est disposée une couche intermédiaire fonctionnelle qui contient un polymère avec des radicaux polymérisables éthyléniquement insaturés.

5. Elément de formation d'images selon l'une des revendications 1 à 4, caractérisé en ce qu'une couche d'accepteur est disposée au-dessus de la couche photopolymérisable (matériau monosheet).

6. Elément de formation d'images selon la revendication 5, caractérisé en ce qu'une couche de pigments est disposée entre la couche photopolymérisable et la couche d'accepteur.

7. Procédé de formation d'images dans lequel un élément de formation d'images selon l'une des revendications 1 à 6 est soumis de manière imagière à une exposition actinique, les parties exposées de façon imagière de la couche photopolymérisable étant durcies et les parties non durcies ou insuffisamment durcies de la couche photopolymérisable étant transférées par chauffage sur une couche d'accepteur.

8. Composé de formule I

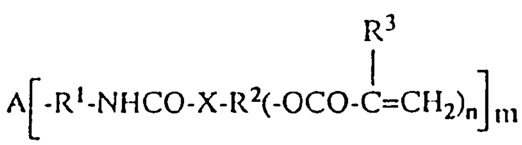

(I),

dans laquelle

A

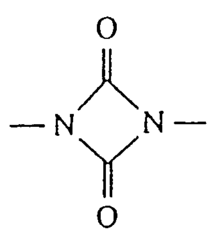

(m = 2)

X désigne -O-;
$R^1$ désigne un radical aliphatique ou cycloaliphatique avec 5 à 8 atomes C;
$R^2$ désigne un radical hydrocarbure à 3 ou 4 liaisons (n = 2 ou 3) avec 3 à 6 atomes C;
$R^3$ désigne H ou un radical méthyle.

9. Composé d'une des formules suivantes:

M-3

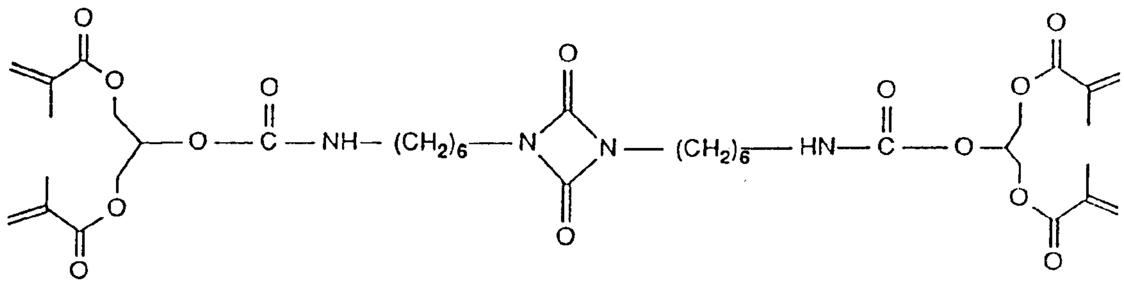

M-4

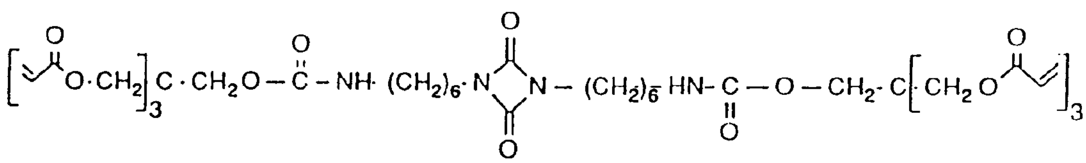

M-5